Europäisches Patentamt

European Patent Office

Office européen des brevets

(11) Publication number: **0 245 052**
**A2**

# EUROPEAN PATENT APPLICATION

(21) Application number: 87303946.5

(22) Date of filing: 01.05.87

(51) Int. Cl.4: **G 01 N 33/564**
**G 01 N 33/68, G 01 N 33/577,**
**A 61 K 39/395**
**// C12P21/00**

(30) Priority: 01.05.86 US 858202

(43) Date of publication of application:
11.11.87 Bulletin 87/46

(84) Designated Contracting States:
AT BE CH DE ES FR GB GR IT LI LU NL SE

(71) Applicant: **IMMUNEX CORPORATION**
**51 University Street Suite 600 600 Immunex Building**
**Seattle Washington 98101 (US)**

(72) Inventor: **Conlon, Paul Jerome III**
**4805 Stanford Avenue Northeast**
**Seattle Washington 98105 (US)**

**Prickett, Kathryn Susan**
**4411 53rd Street Southwest**
**Seattle Washington 98116 (US)**

(74) Representative: **Sheard, Andrew Gregory et al**
**Kilburn & Strode 30, John Street**
**London WC1N 2DD (GB)**

(54) Detection of inflammation and novel antibodies therefor.

(57) An assay, test kit and novel antibodies for use therein are provided for detecting the presence of inflammation caused, for instance, by infectious disease or immune dysfunction, measures the level of IL-1 in body fluid, such as serum. Various assay techniques may be employed including direct assays, competitive assays and double determinant assays. The assay and test kit employs novels polyclonal or monoclonal antibodies specific for unique antigenic sites on the IL-1 protein or IL-1 peptide.

**Description**

## DETECTION OF INFLAMMATION AND NOVEL ANTIBODIES THEREFOR

The present invention relates to immunology, and more particularly to the detection of inflammation by using antibodies to measure the level of interleukin-1 ("IL-1") in body fluids and a test kit for making this measurement.

Many acute and chronic diseases and disorders result in inflammation of blood vessels and adjacent tissue. The diseases and abnormalities may arise from multivarious causes, such as invading pathogens and immune system dysfunctions, such as hypersensitive disorders. The hypersensitive disorders may be based on antibody-mediated immune responses or cell-mediated immune responses, or both. Examples of antibody-mediated responses include anaphylaxis reactions, for example, hay fever, asthma and hives. Other antibody-mediated reactions include Arthus reactions and various immune-complex diseases, for example, glomerulonephritis, viral hepatitis, cryoglobulinemia and bronchopulmonary aspergillosis. Examples of disorders involving cell-mediated hypersensitivity reactions include dermatitis, drug sensitivities and thyroiditis.

Hypersensitivity disorders leading to inflammation that involve both antibody- and cell-mediated responses include autoimmune disorders arising from an autoimmune response. Such responses represent a failure of immunological tolerance to self-components, resulting in the emergence of "forbidden clones" of T and B cells that bear receptors for self-antigens. Autoimmune disorders can affect almost every part of the body, including the thyroid (Hashimoto's thyroiditis), hives (sympathetic ophthalmia), kidneys (Good Pasture's syndrome), red blood cells (autoimmune haemolytic anaemia), liver (primary bilary cirrhosis) and synovial membranes (rheumatoid arthritis).

It is known that inflammation is associated with a complex set of cytologic and histologic reactions in the affected blood vessels and adjacent tissues, including local blood vessel dilation, leading to redness and heat, which permits the influx of plasma, proteins and phagocytic cells into the tissue spaces, thereby resulting in swelling. The local release or activation of other vessel and tissue reactive enzymes and the increased tissue pressure trigger local nerve endings which can result in pain.

Although an accurate test for inflammation would improve the ability to diagnose the various immune dysfunctions discussed above and other inflammation causing disorders and also would be of great assistance in monitoring the effectiveness of treatments chosen for the disorders, in the past it has not been possible to accurately detect inflammation. This may in part be due to the fact that not all of the four cardinal signals of inflammation, i.e., heat, redness, swelling and pain, are always present. In addition, patient assessments of pain are often too subjective to serve as a useful indicator of inflammation. Also, diagnostic procedures previously developed for testing for inflammation have not proven effective. Such procedures have included ascertaining the level of leukocytes, prostaglandins and auto-reactive antibodies in blood, detecting acute phase proteins in serum and plasma, as well as various physical diagnostic procedures aimed at detecting joint mobility. In general, these tests have limited utility due to their unreliability, and in some cases, due to their inconclusive association with a particular disease process. Various specific tests have been developed for individual diseases and disorders that cause inflammation; however, such tests are not useful if the inflammation results from a different disease or disorder.

In addition, prostaglandins are centrally involved in many host immune and metabolic functions. Prostaglandins are intimately involved in "down-regulation" of blood cell generation via their negative feedback effects on colony stimulating factor production. Thus, since prostaglandins naturally control various metabolic functions, measuring for elevated levels of these molecules may not be an accurate indication of an inflammatory reaction.

Applicants have found that the monokine IL-1 is often an early indicator of inflammation and in several ways is centrally involved in the generation of inflammatory responses. For example, IL-1 can be directly pyrogenic, can initiate acute phase protein synthesis, and can even directly cause bone demineralization, as well as cartilage degradation. Moreover, during inflammation, elevated levels of IL-1 can be found in body fluids. For example, elevated levels of IL-1 have been found in rheumatoid synovial fluids obtained from patients suffering from various arthritic conditions. The extent to which IL-1 in body fluid exceeds the normal may be indicative of the extent of inflammation present.

Assays do exist for determining the level of IL-1 in simple solutions. One such assay, developed by applicants and co-researchers, involves ascertaining the capacity of a sample IL-1 to induce proliferation of thymocytes derived from CD-1 mice. Kronheim et al., J. Exp. Med. 161:490-502 (1985). Another assay (denominated "comitogenesis assay") measures the proliferation of murine thymocytes in response to IL-1 and a submitogenic dose of phytohemagglutinin. Mizel et al., J. Immunol. 120(5):1497 (1973). A third assay, that was developed by applicants, employs the ability of IL-1 to convert an interleukin 2 ("IL-2") nonproducer murine tumor cell line, LBRM-33-1A5, into an IL-2 producer, which, in turn, stimulates mitogenesis in the IL-2 dependent continuous T-lymphocyte line (CTLL-2). A limitation of all such assays in that they are based on the biological activity of IL-1. As such, these tests can rarely be standardized from one laboratory/clinic to another. In addition, because they rely on biological assessments of IL-1 function, they are extremely prone to severe inhibition caused by other biological mediators present in serum, plasma, urine, etc., and thus are imprecise and inaccurate with respect to monitoring the presence of IL-1 in such patient fluids. The immunoassays of the invention on the other hand employ antibodies, including novel monoclonal antibodies, to specifically detect the physical presence of IL-1 as opposed to monitoring a later in time biological effect caused by the IL-1, and are, therefore, not affected by the presence of other defined or nondefined mediators in patient fluid samples.

In accordance with the subject invention, inflammation is accurately, rapidly and conveniently detected and quantified by monitoring the physical presence and level of IL-1 in body fluids, for instance, in serum with non-biological assays. Since, as noted above, many infectious diseases and immune dysfunctions result in inflammation, the monitoring of the level of IL-1 in body fluids (eg serum) could be used to diagnose these conditions and also could be employed to choose and monitor the effectiveness of treatments chosen for the disorders.

Measuring the level of IL-1 in body fluids may be especially useful in diagnosing rheumatoid arthritis, which is characterized by symmetric inflammation of the peripheral joints. In addition, IL-1 is a mediator of bone demineralization and cartilage degradation resulting in rheumatoid arthritis. In that over six million Americans suffer from rheumatoid arthritis, the use of the present invention as a diagnostic test for this condition is of substantial utility.

The monitoring of IL-1 in body fluids to detect inflammation has advantages over previously-developed inflammation detection methods discussed above. For instance, applicants have found that elevated levels of IL-1 in body fluids occur early in the inflammation process, so the present invention can provide a sooner indication of inflammation than possible with other detection methods. This is especially true relative to attempts to use the level of prostaglandins to diagnose inflammation. As noted above, one of IL-1's functions is to induce prostaglandin synthesis, thus, measurement of IL-1 provides an assessment of inflammation at a point in time either in inflammation development than is possible if the level of prostaglandins in blood is monitored for this purpose.

In accordance with the present invention, novel polyclonal and monoclonal antibodies are generated for use in the assays of the present invention, discussed below. Applicants and co-researchers have previously discovered that IL-1 is composed of at least two distinct species, designated as IL-1α and IL-1β, March et al., Nature (London) 315L: 641 (1985), incorporated herein by reference. Although not yet fully determined, it is believed that elevated levels of both species occur in body fluids at an early stage of inflammation development. As such, the present invention includes novel antibodies for use in assays for both of these species of IL-1. The polyclonal antibodies are produced by immunizing rabbits, rodents or other animals with IL-1 or IL-1 peptides and then purifying the antibody from the serum of the animal. The novel monoclonol antibodies are produced by injecting IL-1 molecules or peptides into a host animal and then fusing lymphocytes from the immunized animal with neoplastic cells to produce hybridomas capable of expressing a novel monoclonal antibody that specifically binds to the IL-1 molecule.

In another aspect of the present invention, the physical presence and level of IL-1 in body fluids is measured by various immunoassay techniques employing one or more novel antibodies specific for unique antigenic determinants present on IL-1 molecules. In the immunoassays, the reactivity between IL-1 and an antibody specific thereto is determined by observing for the formation of IL-1 antibody complexes, for instance by using fluorescence, radioactive or enzymatic labels. The extent to which such complexes are formed is indicative of the quantity of IL-1 present in the sample, which, in turn, is related to the state and level of inflammation caused by the disorder or disease.

In a more detailed aspect of the present invention, an inflammation condition is directly detected by reacting a specimen of body fluid with a first novel antibody of the present invention that is specific for a first antigenic site uniquely characteristic of the IL-1 molecule, and then measuring the formation of antibody complexes by monitoring the signal produced by a label conjugated either to the first antibody or to a specific binding partner for the first antibody.

The immunoassay may also involve a double determinant assay wherein a specimen of body fluid is contacted with a first novel antibody of the present invention specific for a first antigenic site characteristic of the IL-1 molecule. The first antibody-IL-1 complexes formed thereby are contacted with a second novel anti-IL-1 antibody of the present invention directed at a second unique antigenic site on the IL-1 molecule, which is distinct from the antigenic site reactive with the first anti-IL-1 antibody. After separation of unbound components, the second antibody-IL-1 complexes are measured thereby to determine the quantity of IL-1 "trapped" by the first antibody. This reactivity is determined by measuring the level of signal produced by a label conjugated to the second antibody or to a specific binding partner for the second antibody.

In an additional aspect of the present invention, the IL-1 assay may be in the form of a competitive assay wherein the reactants include a specimen of body fluid, a fixed quantity of a novel anti-IL-1 antibody of the present invention and a fixed quantity of labeled IL-1 antibody. Both the IL-1 present in the sample and the labeled IL-1 are capable of specifically binding with the IL-1 antibody in proportion to their relative amounts present in the reaction. After the bound and unbound components of the reaction are separated, the level of detectable signal produced by the label present in either the bound or unbound components, or both, is measured. The signal level produced by the complexed components of the assay is inversely proportional to the amount of IL-1 present in the sample tested.

A further aspect of the present invention includes diagnostic kits for carrying out the assay procedures of the present invention. The kits include the necessary novel antibody or antibodies of the present invention that are directed against unique antigenic sites present on the IL-1 molecule. The kits further include a signal producing system, for instance composed of a conjugate of a label and a novel anti-IL-1 antibody molecule of the present invention or a conjugate of a label and a specific binding partner for the novel anti-IL-1 molecule. The label may consist of fluorophores, chromophores, radionuclides, color-producing enzymes and paramagnetic metals. The specific binding partner may include polyclonal or monoclonal antibodies reactive with the anti-IL-1 antibody molecule, or any molecule capable of irreversible binding to the antibody molecule itself.

The details of typical embodiments of the present invention will be described in connection with the accompanying drawings, in which:

FIGURE 1 is a competitive assay inhibition curve for the IL-1α specie illustrating the ability of various concentrations of IL-1 in tests samples to inhibit precipitation of radiolabeled IL-1α with a novel anti-IL-1 antibody of the subject invention present in the competitive assay reaction; and,

FIGURE 2 is a competitive assay inhibition curve for the specie IL-1β illustrating the ability of IL-1 in the sample being tested to inhibit precipitation of radiolabeled IL-1β with a novel anti-IL-1 antibody of the subject invention present in the competitive assay reaction.

In accordance with the subject invention, the presence of inflammation causing disorders and diseases by measuring the level of IL-1 in body fluids by employing one or more antibodies specific for unique antigenic determinants present on the IL-1 molecule. Such antigenic sites are particular to the IL-1 molecule and thus distinguish IL-1 from other molecules or cells. The term "inflammation" refers to the cytologic and histologic reactions that occur in affected blood vessels and adjacent tissues of animals in response to injury or abnormal stimulation caused by one or more physical, chemical or biological agents. Such reactions may be local and result in morphological changes, the destruction or removal of the injurious material and repair and healing of the affected tissue. Chronic inflammation is, however, most often associated with persistent infection or a developing disease condition.

In the inflammation detection method of the present invention, the fluid specimen is contacted or otherwise reacted with a novel antibody specifically reactive with IL-1 and then ascertaining the presence or absence of reactivity between the sample and the antibody, preferably by immunoassay techniques. In the various possible immunoassay techniques which might be used, the reactivity between IL-1 and the novel antibody is determined by observing the formation of IL-1-antibody complexes, for instance by fluorescence, radioactive or enzyme methods. The extent to which such complexes are formed is indicative of the level of IL-1 present in the sample which, in turn, is related to the state and extent of inflammation caused by the disorder or disease.

Antibodies

In the subject invention, novel antibodies to IL-1 have been isolated for use in asssays for detecting the presence of IL-1 in body fluid samples. Also, antibodies labeled with a detectable marker are used to identify anti-IL-1 antibodies which have been complexed with the IL-1. Although the preferred type of antibody for use in the anti-IL-1 assays of the present invention is a monoclonal antibody, polyclonal antibodies also may be employed for this use and also as labeled secondary antibodies directed against the anti-IL-1 antibody.

Polyclonal antibodies may be generated against IL-1α and IL-1β by immunizing animals, such as rabbits or guinea pigs, with purified natural and/or recombinant IL-1 of the corresponding species of IL-1. To foster the generation of antibodies by inducing local inflammation, the injected IL-1 may be mixed with an adjuvant, such as complete or incomplete Freund's adjuvant. Preferably, more than one immunization is used, with the immunizations being conducted periodically and in various amounts to induce in vivo generation of antibodies directed against IL-1. Also, rather than injecting the entire volume of IL-1 in one body location, preferably, on each occasion multiple injections are placed subcutaneously and/or intradermally at different locations on the animals. During the incubation regime, the animals are bled and serum samples tested for anti-IL-1 responses with a suitable assay, such as in an enzyme-linked immunoabsorbent assay ("ELISA"). Engvall and Perlman, Immunochem 8: 871-874 (1971). When the serum titer exhibits sufficiently high reactivity to IL-1, the animals are bled and the serum used directly as a source of polyclonal antibody. Alternatively, the antibody can be purified from the serum by standard techniques, such as by protein A affinity chromatography or by affinity purification using an immobilized IL-1 or IL-1 peptide column. The purified Immunoglobin (IgG) fraction can be used as a source of antibody for the assay procedures of the present invention.

As noted above, monoclonal antibodies directed against IL-1 are particularly useful in assaying for IL-1 in body fluids. The anti-IL-1 monoclonal antibodies have advantages over polyclonal antibodies in that such antibodies react with a single antigenic determinant on the IL-1 molecule and can be produced in unlimited supply thereby eliminating the problems of cross-reactivity and differential specificities inherent in polyclonal antibody. However, it has been difficult for researchers to isolate monoclonal antibodies directed against IL-1. One reason for this is that elevated levels of IL-1 have been found to be toxic to mice. Thus, the amounts of IL-1 introduced into mice in an attempt to raise antibodies thereto, must be kept as low levels which in turn reduces the likelihood that antibodies to the IL-1 will be generated. In addition, because IL-1 is markedly pyrogenic, systemic challenge of mice with IL-1 can often lead to extreme fever, which in turn can render an animal immunosuppressed, thereby reducing its chances to mount a humoral, antibodymediated immune response. Nevertheless, applicants have been able to generate a number of novel, monoclonal antibodies to both the IL-1α and IL-1β species of IL-1.

In an exemplary procedure for generating the novel monoclonal anti-IL antibodies of the present invention both purified natural and recombinant IL-1 may be used to immunize an animal, such as a mouse. Preferably, several immunizations are made at periodic intervals. As in the procedure for preparing polyclonal antibodies, to facilitate monoclonal antibody production, preferably the IL-1 is emulsified in an adjuvant, such as Freund's adjuvant, prior to injection. Also, ideally on each occasion multiple injections are placed about the body of the animal.

During the course of the immunization, serum samples from the animal are tested for anti-IL-1 response, for instance by ELISA assay, detailed infra. The animals which test positive are sacrificed and their spleens harvested. Single cell suspensions from the splenocytes are cultured in tissue culture medium supplemented with various additives to expand the number of antibody-producing cells. The spleen cells having chromosomes encoding the base sequence for immunoglobulin molecules reactive with IL-1 are immortalized by fusion with myeloma or lymphoma cells, for instance, from a mouse or human source, to form hybridomas. To facilitate the fusion process, various types of fusing agents may be employed. Such fusing agents may include different types of condensation polymers of ethylene oxide in water, such as polyethylene glycol ("PEG") 1500. Other possible fusing agents include transformed deoxyribonucleic acid ("DNA") viruses, such as Sendai virus or the fusion protein obtained therefrom. For optimum fusion, the quantity and concentration of the fusing agent must be controlled. For instance, if PEG 1500 is used, the fusing agent should comprise about 40% (wt/vol). However, the volume of PEG 1500 may range from 0.5 to 3 milliliters (ml) and the concentration of PEG 1500 may vary from 35% to 60% (wt/vol) of culture medium.

The resulting cells, including the fused hybridomas, are grown in tissue culture medium supplemented with various additives and selected suppressing agents to preclude the growth of unfused myeloma cells, double myeloma hybrids, and unfused spleen cells and double spleen cell hybrids, thereby liberating the anti-IL-1 antibody producing monoclonal cells. Such growth inhibitors or suppressants may include hypoxanthine, aminopterin and thymidine (hereinafter collectively referred to as "HAT"). The hybridoma cells which are generated by this procedure are screened, for instance by ELISA assay, for anti- IL-1 antibody responses. The hybrid cells that test positive are harvested and cloned and subcloned by limited dilution technique, such as detailed in U.S. Patent No. 4,411,993, incorporated herein by reference. In the limiting dilution procedure, anti-IL-1 antibody-producing hybrid cells are individually cultured in vitro in medium together with HAT. The cloning cultures which give rise to hybrid cell growth are screened for reactivity against the IL-1. The cloned hybridomas which test positive are harvested and then cultured in vitro in larger volume for bulk production. Alternatively, the anti-IL-1 antibody may be expanded in vivo by injecting the cloned hybridoma cells into the peritoneal cavity of a suitable mammalian host and thereafter collecting the intraperitoneal ascites which contain high concentrations of anti-IL-1 antibody. The antibodies contained in the ascites fluid can be isolated and concentrated by well-known techniques, such as by differential ammonium sulfate precipitation followed by gel column chromatography. If required, the antibody can be further purified by ion exchange chromatography and/or affinity chromatography based on the ability of the antibody to bind to protein A from Staphylococcus aureis and/or affinity chromatography on an immobilized IL-1 or IL-1 peptide column.

By the above procedure, novel monoclonal antibodies found to be useful in the immunoassay procedures of the present invention were isolated. These unique monoclonal antibodies are of two types, a first type specifically reactive with IL-1α and a second type specifically reactive with IL-1β. One particular novel monoclonal antibody isolated by applicants that is specifically reactive with IL-1α has been designated as 15A4. The 15A4 monoclonal antibody can be classified and characterized by standard procedures, such as by Ouchterlony immunodiffusion and immunoelectrophoresis. The 15A4 monoclonal antibody is of the IgG$_1$ class, reacts specifically with IL-1α and does not bind or cause precipitation of any other lymphokine (i.e., IL-2, IL-1β, granulocyte-macrophage colony stimulating factor ("GM-CSF"), etc.).

One particular novel monoclonal antibody isolated by applicants that is specifically reactive with IL-1β has been designated 7B4. The 7B4 monoclonal antibody also can be classified and characterized by standard procedures such as by Ouchterlony immunodiffusion and immunoelectrophoresis. The 7B4 antibody is of the IgG$_1$ class, reacts specifically with IL-1β and does not bind or cause precipitation of any other lymphokine (i.e., IL-2, IL-1α), GM-CSF, etc.).

Although the specific examples of the novel antibodies of the present invention consist of antibodies of the IgG class, this is not meant to be a limitation. The above antibodies and those having functional equivalency, may be from a murine source, a mammalian source including human, or other source, or other combinations thereof. Also, the antibodies may be of classes other than IgG, for example, IgM, IgA, IgE, including isotypes within these classes. Hybrid antibodies, for instance, composed of F(ab')$_2$ fragments which exhibit functional equivalency to the above-identified monoclonal antibodies, are also within the scope of the present invention. The term "functional equivalency" includes any antibody, isotype, antibody fragment, antibody hybrid or protein product capable of specifically binding to the IL-1 molecule and capable of competing with other antibodies or functional equivalents for binding to the molecule. In other words, the functional equivalent, when combined with a sample containing IL-1 or fragment thereof will bind to the IL-1 or fragment thereof and will block the other antibodies or functional equivalents from binding to the IL-1.

The IL-1 employed to generate the anti-IL-1 antibodies may be derived from both natural and recombinant sources. Natural IL-1 may be produced and purified, for instance, by the procedures set forth in Kronheim et al., _J. Exp. Med._, supra, incorporated herein by reference. As noted above, applicants and co-researchers have previously identified two species of IL-1, denominated as IL-1α and IL-1β. Procedures for the production of recombinant IL-1α and IL-1β are set forth in March et al., _Nature_, supra.

Rather than immunizing animals with the entire natural or recombinant derived IL-1 molecules, peptides composed of a portion of the molecules may be employed. Use of such peptides may facilitate the generation of monoclonal antibodies which are directed at distinct determinant sites on the IL-1 molecule.

Illustrative Assay Methods

Various immunoassay methods may be employed to measure the level of IL-1 physically present in body fluids by use of one or more novel anti-IL-1 antibodies of the present invention. The following are illustrative, but not limiting, examples of such immunoassay methods.

A first or "direct" method includes reacting a fluid sample thought to contain IL-1 with a conjugate of a detectable marker or label and a novel antibody of the present invention specific to an antigenic site on the IL-1 molecule to cause formation of antigen (IL-1)-antibody complexes. The quantity of the IL-1 contained in the sample can be determined by measuring the extent of reactivity of the antibody to the IL-1 by standard signal detection techniques which would be dependent upon the type of label used, as discussed more fully below. In general, the IL-1-antibody complexes are separated from the unbound assay components and then the complexes are qualitatively and/or quantitatively analyzed.

As a variation of the first or direct assay method, rather than conjugating the marker directly to the anti-IL-1 antibody, the marker can instead be conjugated to a suitable, specific binding partner of the novel anti-IL-1 antibody. The binding partner may be a monoclonal or polyclonal antibody directed at a unique determinant site on the anti-IL-1 antibody or an anti-immunoglobulin specific for the anti-IL-1 antibody. In this assay method, the secondary antibody advantageously may be of the type which is more readily conjugated to a label. Also, use of the secondary antibody avoids the possibility that conjugation of the marker to the anti-IL-1 antibody may adversely affect the affinity of the anti-IL-1 antibody. Further, the secondary antibody can be of a polyclonal nature, which generally is easier to isolate than are monoclonal antibodies. In addition, use of a secondary antibody directed to the anti-IL-1 antibody may result in larger complexes which are more easily separated from uncomplexed components of the assay.

Both the presence and quantity of IL-1 in a fluid sample also may be analyzed with a "competitive" immunoassay. In this type of assay, a known amount of a novel anti-IL-1 antibody of the present invention and a known amount of labeled IL-1 are incubated with a sample to be assayed. Since the antibody does not favor either the labeled or unlabeled IL-1, the antibody binds to the labeled and unlabeled IL-1 in proportion to their relative amounts present. Thereafter, the bound components of the assay, IL-1-antibody and labeled IL-1-antibody complexes, are removed from free or unreacted components and then the extent of binding measured by the standard techniques, discussed below. In this type of competitive assay system, a specific concentration of anti-IL-1 antibody is employed. Preferably, a dilution of IL-1 antibody is chosen so that the antibody binds to approximately 50% of the labeled antigen. This results in a bound-to-free ratio of the elements of approximately 1:1. It is to be understood, however, that other dilutions of anti-IL-1 antibody may be chosen without departing from the scope or spirit of the present invention.

In the foregoing competitive assay, prior to the assaying of a particular sample, varying amounts of unlabeled IL-1 are incubated with a fixed quantity of labeled IL-1 and a fixed quantity of the novel anti-IL-1 antibody. The extent to which the labeled IL-1 binds with the anti-IL-1 antibody is then measured for each sample containing the known amount of unlabeled IL-1. From the results of these measurements, a standard curve may be prepared depicting the extent of binding between the labeled IL-1 and the antibody in the presence of a quantity of unlabeled IL-1. Then, when a particular sample containing an unknown amount of unlabeled IL-1 is assayed, the concentration of the unlabeled IL-1 in the sample may be determined from the standard curve once the extent to which the labeled IL-1 binds to the anti-IL-1 antibody is measured.

The present invention also contemplates use of the novel antibodies isolated by applicants in "double determinant" immunoassays for measuring the level of IL-1 in body fluid. In this type of assay a first novel antibody reactive with a unique recognition site on the IL-1 molecule is placed in contact with the sample to be tested. If IL-1 is present in the sample, it binds specifically to the first antibody molecules. After the unbound IL-1 is separated from the bound IL-1, for instance by washing, the first antibody-IL-1 complex is contacted with a second antibody reactive with a different recognition site on the "trapped" IL-1 molecule, which second antibody attaches to the bound IL-1 in a dose-dependent fashion. It will be appreciated that in the double determinant assay it is important that the two anti-IL-1 antibodies are reactive to unrelated epitopes on the IL-1 molecule thereby to avoid stereotypic effects caused by the binding of the two antibodies to the IL-1 molecule.

The second antibody may be labeled so that the extent of binding of the second antibody to the IL-1 can be measured by standard procedures, as discussed more fully below. Rather than labeling the second antibody directly, a binding partner for the second antibody which is conjugated to a marker may be employed. The binding partner may be a secondary antibody that is specific for the second antibody. As noted above, by avoiding the necessity of labeling an anti-IL-1 antibody, the possibility that the labeling may cause a change in the affinity of the antibody is eliminated. Moreover, the antibody employed as the secondary antibody may be chosen on the basis of desirable characteristics, such as ease of labeling, ability to generate and ease of separating bound from unbound antibody. It will be appreciated that in the double determinant assay by using a first novel antibody to "trap" the IL-1 molecule and then a second novel antibody to detect the molecule, a very sensitive immunoassay results.

Although various types of immunoassays have been discussed above, it will be appreciated that numerous variations of the assays may be employed as well as other types of immunoassays. For instance, the labeled or unlabeled IL-1 protein could be replaced with a suitably labeled IL-1 specific peptide with which the antibody(ies) is (are) reactive.

The present invention contemplates the use of various types of insoluble separation matrices or supports in conjunction with the above immunoassays. For example, in the competitive assay the anti-IL-1 antibody, may be covalently or noncovalently bound to suitable supports. The same is true for the anti-IL-1 antibody employed in the double determinant assay. The supports may be composed of the plastic or glass microtiter plate wells or other reaction vessels in which the assay itself is carried out. Alternatively, the support may be in the form of a plastic, cellulose or glass fiber disk, plate or strip which is dipped into or otherwise placed in contact with the IL-1-containing fluid sample. Plastic supports may be of various compositions, such as polyvinyl, polyacrylamide, polystyrene, acrylamide, polypropylene or polycarbonate. The paper support may be composed of nitrocellulose, acetocellulose, or ABM paper. Also, the support may be composed of matrices of various configurations, such as a mesh material or beads of spherical or other shapes which are contained in a reaction vessel. If beads are used, they may be composed of various types of materials, such as agarose, one of the previously-mentioned plastics, glass, cellulose, dextran or Sepharose. Various activating compounds may be employed to covalently bind the antibody to the solid support, which is well known in the art. Such activating agents may include, for instance, cyanogen bromide (CNBr), carbodiimide, glutaraldehyde, polyethylenegycol and tannic acid.

The assays of the present invention are preferably conducted in a liquid medium at moderate pH and temperature. The medium may be of an aqueous nature; however, ideally it is composed of a buffered salt solution media, such as 0.1 molar ("M") Tris buffered saline containing 3% of albumin (ovine, bovine or human). Preferably, the pH of the medium is in the range of about 5-10 and, more preferably, in the range of about 6-9, and ideally about 7.2. The pH is chosen to facilitate specific binding between the IL-1 and the antibody or antibodies, while avoiding any significant negative effect on the signal produced by the marker conjugated to the antibody. To achieve the desired pH and maintain it during the assay procedure various buffers may be employed. Examples of suitable buffers include, for example N-2-hydroxy-ethylpiperazine-N-2-ethane-sulfonic acid ("HEPES"), Tris, borate, phosphate, carbonate and barbital.

As noted above, the present assay may include one or more incubation procedures. For example, in the double determinant assay method, the sample of interest is incubated with a first anti-IL-1 antibody which has been bound to an insoluble support. Thereafter, in a second procedure, the first antibody-IL-1 complex is incubated with a second anti-IL-1 antibody. The length of the incubation period and the incubation temperature will depend, to a large extent, on the binding rate of the IL-1 to the antibody and on the type of label employed. The incubation periods may range from a few minutes to several hours, typically from about 5 minutes to up to 24 hours. The incubation temperatures will generally range from about 1°C to 32°C, and ideally approximately 4°C.

In the present invention, the anti-IL-1 antibody itself or a separate, secondary antibody directed to the anti-IL-1 antibody may be conjugated with a detectable marker to produce a signal related to the presence of IL-1 in the test sample. The detectable marker can be selected from among fluorophores, colored dyes, enzymes, chromophores, coenzymes, chemilluminescent materials, enzyme inhibitors, paramagnetic materials such as gadolinium, ferritins and radionuclides that are known in the art. Illustrative but nonlimiting examples of particular enzymes which might be employed include horseradish peroxidase, alkaline phosphatase, and B-galactosidase. Illustrative examples of colored dyes include without limitation amido black and eosin. Illustrative fluorescent compounds include without limitation fluorescein, isothiocyanate, dansyl, propidium iodine as well as phycophores, such as phycoerythrin.

The detectable marker may also be composed of a radioactive isotope. The technique used for labeling the antibody varies with the type of radioactive isotope employed. For instance, labeling can be accomplished by replacing one of the atoms of the antibody molecule with a corresponding radioactive isotope. As a specific example, a hydrogen atom could be replaced with tritium ($^3$H); a carbon atom could be replaced by carbon-14 ($^{14}$C); or, a strontium atom replaced with strontium-38 ($^{38}$Sr). In an alternative labeling process, rather than replacing the atoms of the antibody with a radioactive isotope, an isotope may be added to the antibody molecule. Such radioactive isotopes in common use include, for instance, iodine-125 ($^{125}$I) and iron -59 ($^{59}$Fe).

It will be appreciated that the particular marker or label employed depends on various factors, such as the particular type of immunoassay being used and the biological and biochemical characteristics of the anti-IL-1 antibody or secondary antibody being labeled. Whatever type of marker is employed, it, of course, should not cause any significant change in the specificity between the labeled antibody and its specific recognition site.

After each of the various incubation steps in the assay of the present invention, the complexed or bound components of the assay typically are separated from the unbound components, non-complex IL-1, excess anti-IL-1 antibodies and secondary antibodies. The methods may include simply washing with, for instance, a saline solution alone or combined with centrifugation. The separation may include ultrafiltration, dialysis or salt precipitation. Other separation procedures may be based on differential biochemical migration, for instance, chromatography, electrophoresis, chromatoelectrophoresis and gel filtration. The particular type of separation method(s) employed will depend upon the specific immunoassay procedure used and the characteristics of the assay reagents.

Diagnostic Kit

The present invention also includes a diagnostic kit for carrying out the IL-1 assays disclosed above to detect the presence of inflammation. The particular components of the kit correspond to the particular immunoassay procedure being employed. In perhaps its simplest embodiment, the diagnostic kit may include a novel polyclonal or monoclonal antibody of the present invention directed against IL-1 which has been conjugated with a suitable marker capable of producing a detectable signal. To carry out the assay, the test sample is placed in contact with the antibody-marker conjugate. Thereafter, the complexed components are separated from the free components of the assay and then the signal produced by the marker is detected and quantified in either the bound or free components of the immunoassay reaction. As noted above, the assay components may include an insoluble matrix on which the antibody is covalently or noncovalently coupled, buffers to maintain the desired pH of the immunoassay reaction and binding media to dilute the fluid sample. The kit may also include reagents required for the marker to produce a detectable signal, such as an appropriate enzyme reagent for ELISA assay, or agents to enhance the detectable signal.

In another illustrative, but nonlimiting example, the diagnostic kit may include a novel polyclonal or monoclonal antibody directed against IL-1 and a secondary antibody directed against the anti-IL-1 antibody, which second antibody is conjugated to a suitable marker capable of producing a detectable signal. As in the embodiment of the assay kit discussed above, this kit embodiment also may include other additional components. To carry out the assay, a test sample is placed in contact with the anti-IL-1 antibody and then the complexed components separated from the free components. Thereafter, the complex components are placed in contact with the labeled secondary antibody which specifically couples with the anti-IL-1 antibody bound to the IL-1. After the unbound secondary antibody is separated from the complexed components of the assay, the signal produced by the label is measured in either the bound or free components of the assay reaction.

In a further illustrative embodiment of the present invention, the diagnostic kit may include the components necessary to carry out the double-determinant assay procedure described above. This particular kit composition includes novel first and second antibodies directed against separate determinant sites on the IL-1 molecule. Although not essential, preferably the first antibody is covalently or noncovalently coupled to a support matrix. The second anti-IL-1 antibody may be conjugated with a suitable marker capable of producing a detectable signal, or alternatively a third labeled secondary antibody directed against the second anti-IL-1 antibody may be employed. Again, as noted above, the kit may include various additional components to optimize or facilitate the assay procedure.

Measurement of Reactivity

The process and equipment employed for measuring the binding between the IL-1 present in the sample and the antibodies directed thereto depends upon the type of detectable marker employed. If the marker is composed of an enzyme or dye, the color produced by the assay may be measured at the appropriate wavelength by suitable automated equipment, for instance with a Multiskan plate reader (Flow Laboratories, McLean, VA). If a fluorescent marker is used, the reactivity of the assay components may be analyzed by, for instance flow microfluorimetry, using a FACS 440 microfluorinater (Becton Dickinson, Palo Alto, CA) or a Screen Machine (Pandex, Inc., Mundelein, IL). If a radioactive marker is employed, a gamma counter may be used for isotopes that emit gamma rays or a liquid scintillation counter used for isotopes that emit beta rays. Such counters are standard articles of commerce.

The processes and products of the present invention are further illustrated by the following alphabetically denominated examples of particular procedures used in the present invention and then followed by illustrative numbered examples. The following examples are presented to illustrate the present invention and to assist one of ordinary skill in making and using the same. The examples are not intended in any way to otherwise limit the scope of disclosure or the protection granted by Letters Patent hereon.

EXAMPLE A

ELISA Assay

As noted above, polyclonal antibodies, hybridoma supernatants and monoclonal antibodies may be tested for anti-IL-1 responses in an ELISA assay. In the assay, purified, natural or recombinant IL-1 is diluted to a concentration of approximately 1 nanogram per microliter (ng/ul) in 0.1 M Tris buffered saline containing bovine serum albumin (T-BSA). Approximately 20 microliters (ul) of this solution is applied to each well of 200 ul nitrocellulose ELISA plates by using a stepper repetitive pipette. The fluid from the solution is allowed to evaporate thereby to non-specifically adhere the IL-1 to the nitrocellulose.

As an alternative to the foregoing, the wells of the dish can be coated with IL-1 diluted with T-BSA as above. After an incubation period of 50 minutes at 37°C, the excess fluid is removed through the nitrocellulose filter by vacuum.

Next, the unreacted sites of the well are blocked with 100 ul of T-BSA for 60 minutes at 37°C. The T-BSA thereby prevents non-specific adherence of the antibody of interest to the wells. After this incubation period the wells are washed three times with phosphate (0.05M) buffered saline (0.15M) ("PBS") at pH 7.2 by suction.

Samples to be tested (animal serum containing polyclonal antibodies, monoclonal antibodies or hybridoma supernates) at volumes of 50-100 ul are added to the wells and incubated for approximately 60 minutes at 37°C. After incubation, the antibody solutions are removed and each well washed three to five times with saline by suction. Next, approximately 50-100 ul volumes of enzyme-labeled anti-immunoglobulin antibody is added to each well, for instance, an alkaline phosphatase conjugated secondary antibody. If the assay is being employed to analyze hybridoma supernates for anti-IL-1 reactivity, preferably the alkaline phosphatase conjugated reagent is a goat anti-mouse IgG antibody (Sigma Chemical Company, St. Louis, MO) employed at approximately a 1:500 dilution in 3% T-BSA. If the assay is being used to detect polyclonal antibodies to IL-1, for instance from rabbit sources, preferably the alkaline phosphatase conjugated reagent is a goat anti-rabbit IgG antibody (Sigma Chemical Company), employed at approximately a 1:200 dilution in 3% T-BSA.

After a 30 minute incubation period with the appropriate alkaline phosphatase coupled antibody, each well is washed three to five times with saline. Next, approximately 100 ul of a colorless alkaline phosphatase substrate is added to each well. One such preferable substrate is paranitrophenyl phosphate (Sigma Chemical Company). This substrate is prepared at a strength of approximately 1 mg/ml with substrate buffer composed of 0.1 M glycine (pH 10.4), 1 mM zinc chloride, and 1 mM magnesium chloride. After 30 minutes of incubation, 50-75 ul aliquots are removed from each well and transferred into and ELISA plate. If anti-IL-1 antibody has bound to the IL-1 coupled to the plate, a colored product is formed. The optical density of the color can be ascertained by measuring the absorption of the color at 405 nanometers with a Multiskan plate reader (Flow Laboratories). The value of the optical density measured is directly proportional to the quantity of IL-1 antibody in the well sample.

## EXAMPLE B

### Radiolabeling of IL-1α Specie

The α specie of IL-1 was radiolabeled with $^{125}$I using a modified chloramine-T method. In the method 13 ug ($7.43 \times 10^{-10}$ moles) of IL-1α were incubated with 1.5 microcurrie ("uCi") ($6.10^{-9}$ moles) of Na[125]I (NEN) and $4 \times 10^{-9}$ M of chloramine-T (Sigma Chemical Company) in 65 ul of 0.05 M sodium phosphate buffer (pH 7.0) on ice for 30 minutes. The Na[125]I-IL-1α conjugate was separated from unincorporated Na[125]I on a 1 ml bed volume Biogel P6 column (Biorad, Richmond, CA). Through sodium dodecyl sulfate polyacrylamide gel electrophoresis (SDS-PAGE), the radiolabeled IL-1α was found to contain a single $^{125}$I polypeptide having a molecular weight of approximately 17,500 daltons. The radiolabeled Il-1α was also found to be more than 95% TcA precipitable, more than 95% bindable to IL-1-receptor bearing cells, and exhibited a specific activity in the range of from 1 to $5 \times 10^{15}$ counts per minute per millimole (CPM/mM).

## EXAMPLE C

### Radiolabeling of IL-1β Specie

The β specie of IL-1 was labeled using Di-iodo ($^{125}$I)-Bolton-Hunter reagent (New England Nuclear, Boston, MA). In the labeling procedure, 200 ng of IL-1β in 20 ul of 0.2 M sodium borate and 0.15 M NaCl buffer (pH 8.5) was mixed with 5 ul of 0.1 M sodium borate and 0.7 M NaCl buffer (pH 8.5) and then added to a vial in which 100 ul of benzene containing 1 mCi (0.23 nanomoles) of Bolton-Hunter reagent had been evaporated using a gentle stream of dry nitrogen. After the reaction was allowed to proceed for one hour on ice, it was terminated by adding 5 ul of 1 M glycine ethyl ester. 30 ul of 2% gelatin in PBS, pH 7.2, was added as a carrier and the radiolabeled IL-1 was separated from free Bolton-Hunter reagent by chromatography on a 1 ml bed volume column of Biogel P6 (Biorad) packed in a pasture pipette. The column bed material was blocked with bovine serum albumin ("BSA") and subsequently washed with 20 ml of PBS to remove unbound BSA prior to use. 100 ul fractions were collected and the fractions containing protein-bound radioactivity were pooled.

Because of the small amounts of IL-1β available, direct estimates of the recovery following the radiolabeling procedure were not possible. In order to estimate recovery, more controlled experiments were conducted in which IL-1β (200 nanograms) was mixed with $^{125}$I-IL-1 ($3 \times 10^4$ cpm) and placed through the iodination procedure, but with the omission of the Bolton-Hunter reagent. In five replicate experiments, $54 \pm 8\%$ of the counts were recovered after gel filtration. In subsequent iodinations, this percentage was taken as the recovery of IL-1β protein. Using this estimate, the specific activity of the radiolabeled IL-1β preparations was in the range of about $2\text{-}5 \times 10^{15}$ CPM/mM based on a molecular weight of the IL-1β peptide of about 17,500 daltons.

## EXAMPLE 1

### Production of Polyclonal Anti-IL-1β Antibody

Young rabbits were immunized subcutaneously and intradermally in the back with purified natural and recombinant IL-1β. An initial immunization was conducted with a 200 ug dose mixed with complete Freund's adjuvant. Subsequent immunizations with 100 ug doses mixed with incomplete Freund's adjuvant were conducted monthly for four months.

Rather than injecting the entire volume of a particular immunization in one location, on each occasion multiple injections are placed subcutaneously in the backs of the rabbits.

During the immunization regime, serum samples from the rabbits were tested for anti-IL-1 responses in the ELISA assay discussed above. When the rabbit serum titer was high enough to react with the IL-1 at dilutions of 1:100 or greater, antisera was taken from the rabbits on a bimonthly basis, beginning one month after the last immunization and used as one source of antibody for the assays of the present invention.

EXAMPLE 2

Production of Polyclonal Antibody Directed Against IL-1α Specie

Polyclonal antibody directed against the specie of IL-1 was also generated in rabbits using substantially the same procedure discussed above in Example 1 concerning the production of polyclonal antibody directed against the β specie of IL-1 with the following variations. In the procedure used to generate the anti-IL-1α polyclonal antibody, the rabbits were initially immunized with 250 ug of purified IL-1α in complete Freund's adjuvant. Two months later the animals were boosted with immunizations of 170 ug of purified IL-1α in incomplete Freund's adjuvant. After the rabbit serum titer had reached a sufficient level (i.e., a reactive titer in excess of 1:100), antisera was taken from the animals on a bimonthly basis, and used as one source of antibody for the assays of the present invention.

EXAMPLE 3

Production of Monoclonal Antibody to IL-1

BALB/c mice were immunized subcutaneously and intradermally with 150 ug of IL-1α peptide. The peptide for such immunization was composed of the C-terminus of the IL-1α molecule consisting of amino acids 256-271 in March et al., Nature, supra. Prior to immunization, the IL-1 peptide was prepared as an emulsion with 150 ug of IL-1 peptide in 0.5 ml of PBS and 0.5 ml of complete Freund's adjuvant (Difco Laboratories, Detroit, MI). After the initial immunization, the mice were challenged monthly for three months 75 ug of IL-1α peptide in incomplete Freund's adjuvant.

After the second immunization, and each immunization thereafter, serum was collected from the mice and tested for anti-IL-1α antibody response by use of the ELISA assay as detailed above. After the injections were completed, mice with high serum titer antibody to IL-1α (greater than 1:100) were sacrificed and their spleens harvested. Single cell suspensions were prepared therefrom. The spleen cells were cultured in Click's medium (Altick Associates, Hudson, WI). The medium was supplemented with 10% (vol./vol.) heat-inactivated fetal calf serum (FCS), 300 ug/ml of fresh L-glutamine, 50 ug/ml of gentamycin, 50 U/ml of penicillin, 50 ug/ml of streptomycin, 25 mM HEPES buffer, 300 ug/ml sodium pyruvate and 16 mM $NaHCO_3$ (complete Click's medium).

The spleen cells that developed from the single suspensions were fused with NS1 murine myeloma cells. Fusion was achieved by mixing approximately $20 \times 10^6$ spleen cells with approximately $10 \times 10^6$ NS1 murine myeloma cells in 15 ml conical centrifuge tubes. The cell mixture was pelleted by centrifugation for 10 minutes at 250 X g and the supernate discarded. One ml of a solution of 40% (wt./vol.) of PEG diluted in complete Click's medium was then added to the cell pellet in a dropwise manner. Thereafter, 10 ml of complete Click's medium was added to the centrifuge tube over a 2 minute period and the cell pellet gently resuspended. Next, the mixture was centrifuged for 5 minutes at 250 X g and then the supernate discarded to complete the fusion process.

The resulting cell pellets were resuspended in 40 ml of complete Click's medium. the unfused myeloma driver cells (NS1), double NS1 hybrids, unfused spleen cells and double spleen cell hybrids were prevented from proliferating by the addition to the medium of approximately 1.35 mg/ml of hypoxanthene, 0.00176 mg/ml of aminopterin and 0.388 mg/ml of thymidine (complete Click's HAT medium). The entire suspension was then divided into 200 ul aliquots in flat-bottom microtiter plates (No. 3596 Costar, Inc., Cambridge, MA). The cultures were all maintained at approximately 37° in a humidified atmosphere of 7% $CO_2$ in air. After from 7 to 10 days of culture, supernates from wells containing viable hybrid cells were tested by ELISA assay for the presence of anti-IL-1 antibody. The hybrid cells that tested positive were harvested and cloned by limiting dilution technique. The cloning cultures that gave rise to hybrid cell growth were screened by ELISA assay.

Through the above procedure, novel antibodies to IL-1α were developed. Examples of such antibodies that were found to be useful in the present invention are designated as 7G9-C1, 7H4-G1, 13D12-B2, 14D1-13H4, 15A4-3C12, 15C2-D6 and 16H11-H10. The hybridoma cell lines that produce these monoclonal antibodies are given the same designation. A sample of the 15A4-3C12 cell line has been deposited with the ATCC under Accession No. HB 9084.

The anti-IL-1 antibody 15A4-3C12 was expanded in vivo by intraperitoneal injection of BALB/c mice with approximately $2 \times 10^6$ hybridoma cells which produced this antibody. One week prior to hybridoma cell injection, recipient BALB/c mice were given 1.0 ml of pristane intraperitoneally as an ascites inducing irritant. From 8 to 14 days after hybridoma injection, intra-peritoneal ascites were collected and then the 15A4-3C12 monoclonal antibody clarified by centrifugation for 10 minutes at 1,000 X g.

EXAMPLE 4

Production of Monoclonal Antibody to IL-1β

The procedure delineated in Example 3 was employed to generate a series of monoclonal antibodies reactive with human IL-1β. Mice used as a source of spleen cells for generation of monoclonal antibodies directed against IL-1β were first immunized subcutaneously and intradermally with 200 g of human IL-1β in 0.5 ml of PBS, and 0.5 ml of complete Freund's adjuvant. After the initial immunization, the mice were challenged monthly for three months with 75µg of IL-1β in incomplete Freund's adjuvant. These monoclonal antibodies have been designated as: 3A4-10G, 3H3-H9, 4C12-3F, 5A11-4B, 6B11-C1 and 7B4-2B. A sample of the 7B4-2B cell line is deposited with the ATCC under Accession No. HB 9085.

## EXAMPLE 5

### Characterization of 7B4-2B and 15A4-3C12 Antibodies

Both 15A4-3C12 and 7B4-2B and IgG₁ antibodies. The 15A4-3C12 antibody reacts specifically with IL-1α and not IL-1β, and more specifically is directed to an antigenic determinant contained in the C-terminal 15 amino acids of the IL-1α molecule. The 7B4-2B antibody reacts specifically with IL-1β and not IL-1α. Neither antibody demonstrated specific reactivity to other lymphokines (IL-2, GM-CSF, IL-3, interferon, etc.). Both antibodies can be purified from ascites either via affinity chromatography using Protein A Sepharose (Sigma Chemical Co.), or by sequential gel exclusion and ion exchange chromatography.

## EXAMPLE 6

### Competitive Assay for IL-1α and IL-1β

Competitive radioimmunoassays (RIA) were performed in 600 ul RIA tubes (Sarstedt, Inc., Princeton, N.J., Cat. #73.1055) in final volumes of 150 ul. The following immune reaction components were placed in each of the RIA tubes: (1) 50 ul of either immune sera from Examples 1 or 2 above, 50 ul of hybridoma supernatant from Examples 3 or 4, ascites from Examples 3 or 4, or purified anti-IL-1 IgG in PBS as prepared in Examples 3 or 4 above, diluted in binding media (500 ml Roswell Park Memorial Institute "RPMI") 1640 medium, 50 ml of 0.2 M HEPES [pH 7.2], 12.5 g BSA [Sigma Chemical Company] and 0.5 g sodium azide [pH 7.7]); (2) 50 ul of 20% (vol./vol.) solution of Protein A Sepharose (Sigma Chemical Company) in PBSTA (PBS, 1% Triton X100, 5% BSA [Sigma Chemical Company] and 0.2% sodium azide [pH 7.0]); (3) 25 ul of binding media containing radiolabeled IL-1α or IL-1β (45,000 cpm); and, (4) 25 ul of binding media containing increasing concentrations of unlabeled IL-1α or IL-1β. The RIA tubes were placed in a Mini-Orbital shaker (Bellico Vineland, N.J.) and shaken for 16 hours at 4°C (setting of shaker at 5-1/2). Thereafter, the samples were washed once with 200 ul of PBS and then twice with 400 ul of PBS by centrifugation of the samples in 96 well RIA tube holders at 500 RPM for 10 minutes (Sorvall RT6000 refrigerated centrifuge) followed by vacuum aspiration of the supernatant. The RIA tubes were then placed into Dispo borosilicate glass culture tubes (American Scientific Products, McGaw Park, IL, Cat. #T1290-3) and the radioactivity of the immune complex measured with a Packard Multi-Prias 4 gamma ray counter (United Technologies, Downers Grove, IL).

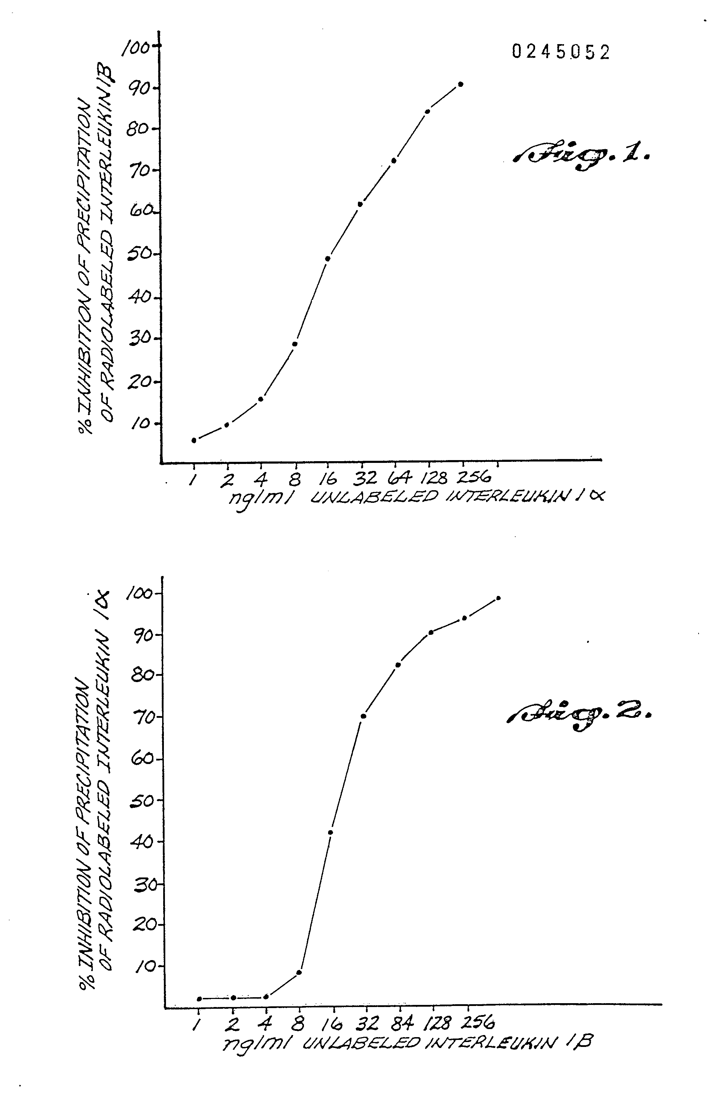

From the radioactivity emission level measurements, standard inhibition curves were plotted in terms of percent of inhibition of binding of the radiolabeled IL-1α or IL-1β to their respective antibodies caused by varying quantities of unlabeled IL-1α or IL-1β. Inhibition curves for the anti-IL-1α antibody and the anti-IL-1β antibody are shown in FIGURES 1 and 2, respectively. Nonspecific background levels of binding were determined by conducting the competitive assays by replacing the immune serum with 50 ul of non-immune rabbit serum diluted with binding media. In the case of monoclonal antibodies (from ascites fluid or purified IgG₁ diluted in PBS) a control solution of PBS, pH 7.2, serves as an appropriate sample to determine nonspecific background levels of reactivity. Samples containing unknown amounts of IL-1α or IL-1β can now be measured for percent of inhibition of the radiolabeled reaction, and from this measurement the quantity of IL-1α or IL-1β present can be determined by using the curves set forth in FIGURES 1 and 2. The samples can be obtained from body fluids, such as serum, saliva, urine, plasma and synovitis fluids. Samples taken from healthy volunteers can be used to establish normal levels of IL-1 in test fluids when inflammation is not present. This provides a reference level for use in quantifying the degree of inflammation present based on elevated levels of IL-1 detected in the fluid samples assayed.

## EXAMPLE 7

### Determination of Inflammation with Double Determinant Assay

A first monoclonal antibody is diluted to a concentration of approximately 10 ug/ml in PBS. Approximately 100 ul of this solution is placed in a reaction vessel composed of a 96 well 200 ul nitrocellulose ELISA plate. The fluid from the solution is allowed to evaporate during an incubation process thereby to nonspecifically adhere the first monoclonal antibody to the plate. Thereafter, the plate is washed three times with approximately 100 ul of PBS, an additional 100 ul PBS containing 3% BSA (by weight) is added and then the plate incubated at 32°C for 60 minutes to block the remaining sites on the bottom of the plate wells that have not already bound to the first monoclonal antibody. After this additional incubation, the PBS solution is removed either by a vacuum or decanted by washing three times with 100 ul of PBS. The plate is now ready for use in the double determinant immunoassay procedure. It will be appreciated that the plate or other appropriate vessel, such as a tube, plastic well plate, etc., may be employed as part of a kit for clinical and other uses.

Samples of body fluid in a volume of 50 ul are added to the reaction plate well and then incubated for 60 minutes at 32°C. After incubation, the fluid is removed and the plate wells repeatedly washed with 100 ul of PBS. Thereafter, 100 ul of a second monoclonal antibody directed against IL-1, is added to the reaction well after having been diluted with PBS to a concentration of approximately 100 ug/ml. The second monoclonal antibody is reactive with a different epitope on the IL-1 molecule than the epitope with which the first antibody is reactive. After incubation for approximately 60 minutes at 32°C, the second antibody solution is removed and the vessel repeatedly washed with 100 ul of PBS to remove the second antibody. Thereafter, approximately 50-100 ul of an alkaline phosphatase conjugated secondary IgG antibody specific for the second monoclonal antibody is added to the reaction vessel. The secondary antibody is used at approximately a 1:500 dilution in PBS containing approximately 3% BSA. After an incubation period of 30 minutes at 32°C, the vessel is repeatedly washed with either normal saline (approximately 0.9% wt/vol.) or by immersion in tap water. Next, approximately 100 ul of paranitrophenyl phosphate substrate (Sigma Chemical Company) is added to the vessel. The substrate is prepared at a strength of approximately 1 mg/ml together with approximately 0.1 M glycine (pH 10.4), 1 mM zinc chloride and 1 mM magnesium chloride. If IL-1 is present in the sample being tested, a colored product is formed. The optical density of the color is then measured at 405 nanometers with a Multiskan plate reader. The value of the optical density measured is directly proportional to the quantity of IL-1 in the sample which in turn is indicative of the inflammation condition existing in the source of the sample.

As will be apparent to those skilled in the art to which the invention is addressed, the present invention may be embodied in forms other than those specifically disclosed above without departing from the spirit or essential characteristics of the invention. The particular embodiments of the present invention, described above, are therefore to be considered in all respects as illustrative and not restrictive. The scope of the present invention is as set forth in the appended claims, rather than being limited to the foregoing embodiments of the present invention.

**Claims**

1. A method for detecting inflammation mediated by interleukin 1 ("IL-1") in a subject, comprising:

(a) reacting a specimen of body fluid suspected of containing IL-1 of the subject with a first antibody specific for a first antigenic site characteristic of IL-1; and,

(b) detecting the reaction between the first antibody and the IL-1 in the specimen.

2. A method for detecting an auto-immune disease condition mediated by the presence of IL-1 in the subject being tested, comprising:

(a) contacting a specimen sample taken from the subject with a first antibody under conditions for binding the first antibody to IL-1 present in the specimen sample; and

(b) determining the presence of immune complexes of the first antibody.

3. A method as claimed in Claim 1 or 2, wherein the first antibody is a monoclonal antibody selected from the group consisting of 15A4-3C12 and 7B4-2B, or a functional equivalent thereof.

4. A method as claimed in any one of Claims 1 to 3, wherein the extent of the reaction between the first antibody and the IL-1 is determined by:
contacting the specimen with a second anti-IL-1 antibody directed at a second antigenic site on the IL-1 molecule which is structurally distinct from the antigenic site reactive with the first anti-IL-1 antibody; and,
measuring the reaction between the second antibody and the IL-1-first antibody complex.

5. A method as claimed in any one of Claims 1 to 4, wherein:
the specimen is also reacted with a known quantity of labelled IL-1; and,
the IL-1-first antibody reaction is measured by measuring the reactivity between the first antibody and the labelled IL-1.

6. A diagnostic kit for testing for the presence of an inflammation condition in a subject, comprising:
a first antibody specific for an antigenic site on a protein or peptide characteristic of IL-1 and useful binding fragments of said first antibody; and,
a signal producing system capable of producing a detectable signal associated with the binding of the first antibody to its corresponding antigenic site on the protein.

7. A kit as claimed in Claim 6, wherein the signal producing system comprises:
a second antibody specific for a second antigenic site on the protein characteristic of IL-1, the second antigenic site being structurally distinct from the first antigenic site, and useful binding fragments of the second antibody; and,
a label capable of producing a detectable signal related to the binding of the second antibody to the protein characteristic of IL-1.

8. A kit as claimed in Claim 6 or 7, wherein the signal producing system comprises:
a second antibody specific for a second antigenic site on the protein characteristic of IL-1, the second antigenic site being structurally distinct from the first antigenic site, and useful binding fragments of the second antibody;
a specific binding partner to the second antibody; and,
a label conjugated to the specific binding partner.

9. A monoclonal antibody having the identifying characteristics of 15A4-3C12 (ATCC HB 9084).

10. A monoclonal antibody having the identifying characteristics of 7B4-2B (ATCC HB 9085).

11. The use of an antibody against interleukin 1 ("IL-1") in the preparation of an agent for the detection of inflammation.

12. The use of an antibody against interleukin 1 ("IL-1") in the preparation of an agent for the detection of an auto-immune disease.

CLAIMS FOR THE FOLLOWING CONTRACTING STATES: AT, ES and GR.

1. A method for detecting inflammation mediated by interleukin 1 ("IL-1") in a subject, comprising:

(a) reacting a specimen of body fluid suspected of containing IL-1 of the subject with a first antibody specific for a first antigenic site characteristic of IL-1; and,

(b) detecting the reaction between the first antibody and the IL-1 in the specimen.

2. A method for detecting an auto-immune disease condition mediated by the presence of IL-1 in the subject being tested, comprising:

(a) contacting a specimen sample taken from the subject with a first antibody under conditions for binding the first antibody to IL-1 present in the specimen sample; and

(b) determining the presence of immune complexes of the first antibody.

3. A method as claimed in Claim 1 or 2, wherein the first antibody is a monoclonal antibody selected from the group consisting of 15A4-3C12 and 7B4-2B, or a functional equivalent thereof.

4. A method as claimed in any one of Claims 1 to 3, wherein the extent of the reaction between the first antibody and the IL-1 is determined by:

contacting the specimen with a second anti-IL-1 antibody directed at a second antigenic site on the IL-1 molecule which is structurally distinct from the antigenic site reactive with the first anti-IL-1 antibody; and,

measuring the reaction between the second antibody and the IL-1-first antibody complex.

5. A method as claimed in any one of Claims 1 to 4, wherein:

the specimen is also reacted with a known quantity of labelled IL-1; and,

the IL-1-first antibody reaction is measured by measuring the reactivity between the first antibody and the labelled IL-1.

6. A diagnostic kit for testing for the presence of an inflammation condition in a subject, comprising:

a first antibody specific for an antigenic site on a protein or peptide characteristic or IL-1 and useful binding fragments of said first antibody; and,

a signal producing system capable of producing a detectable signal associated with the binding of the first antibody to its corresponding antigenic site on the protein.

7. A kit as claimed in Claim 6, wherein the signal producing system comprises:

a second antibody specific for a second antigenic site on the protein characteristic of IL-1, the second antigenic site being structurally distinct from the first antigenic site, and useful binding fragments of the second antibody; and,

a label capable of producing a detectable signal related to the binding of the second antibody to the protein characteristic of IL-1.

8. A kit as claimed in Claim 6 or 7, wherein the signal producing system comprises:

a second antibody specific for a second antigenic site on the protein characteristic of IL-1, the second antigenic site being structurally distinct from the first antigenic site, and useful binding fragments of the second antibody;

a specific binding partner to the second antibody; and,

a label conjugated to the specific binding partner.

9. A process for the preparation of a monoclonal antibody having the identifying characteristics of 15A4-3C12 (ATCC HB 9084) comprising causing a culture of hybridoma cells capable of expressing antibodies having such characteristics to express the antibodies.

10. A process for the preparation of a monoclonal antibody having the identifying characteristics of 7B4-2B (ATCC HB 9085) comprising causing a culture of hybridoma cells capable of expressing antibodies having such characteristics to express the antibodies.

11. The use of an antibody against interleukin 1 ("IL-1") in the preparation of an agent for the detection of inflammation.

12. The use of an antibody against interleukin 1 ("IL-1") in the preparation of an agent for the detection of an auto-immune disease.

0245052

Fig. 1.

% INHIBITION OF PRECIPITATION OF RADIOLABELED INTERLEUKIN IB (y-axis)

ng/ml UNLABELED INTERLEUKIN Iα (x-axis)

Fig. 2.

% INHIBITION OF PRECIPITATION OF RADIOLABELED INTERLEUKIN Iα (y-axis)

ng/ml UNLABELED INTERLEUKIN IB (x-axis)